# EUROPEAN PATENT APPLICATION

(11) **EP 4 656 649 A1**
(43) Date of publication of application: **03.12.2025**
(21) Application number: 24769858.2
(22) Date of filing: 08.03.2024
(51) Int. Cl.: C07K 14/195, A61K 39/02, A61K 39/00, A61P 17/10, A61P 17/00, C12N 15/31

(54) **ANTI-ACNE RECOMBINANT PROTEIN VACCINE, PREPARATION METHOD, AND USE**

(30) Priority: 10.03.2023 CN 202310228339
(71) Applicant: WestVac Biopharma Co., Ltd., Chengdu, Sichuan 610000 (CN)
(72) Inventor: LI, Jiong, Chengdu, Sichuan 610065 (CN); HUANG, Nongyu, Chengdu, Sichuan 610065 (CN); WEI, Yuquan, Chengdu, Sichuan 610065 (CN)
(74) Representative: Gulde & Partner
(86) International application number: PCT/CN2024/080674
(87) International publication number: WO 2024/188165

(57) **Abstract**

Provided are an anti-acne recombinant protein vaccine, a preparation method, and a use. In order to solve the current problem that there is still a lack of a drug for effective prevention and treatment for acne diseases, a recombinant protein vaccine targeting a CAMP virulence factor of Cutibacterium acnes is provided, which is mainly used for inducing an immune response in vivo, such as the production of antibodies, to neutralize the CAMP virulence factor and inhibit Cutibacterium acnes-induced skin inflammation, so as to have the effect of preventing and treating acne.

## Description

### TECHNICAL FIELD

The present invention belongs to the technical field of biomedicine, and in particular, to an anti-acne recombinant protein vaccine, a preparation method therefor, and an application thereof.

### BACKGROUND

Acne is the eighth largest epidemic in the world, affecting more than 640 million people worldwide. According to Chinese Guideline for Diagnosis and Treatment of Acne (2019 Edition), the cross-sectional statistics show that the incidence of acne among the Chinese population is 81%, but more than 95% people will develop acne to varying degrees. The onset of acne usually occurs with hormonal changes during adolescence, and the condition affects about 85% teenagers and young people aged 12 to 25. Acne can also persist into adulthood or develop during adulthood. Acne lesions and/or scars may persist in adults. Although acne is not a life-threatening disease, psychosocial burden resulting from acne may be high and underestimated. A recent study shows that acne will have a negative impact on the quality of life of 90% patients. It is noteworthy that more than half of acne patients exhibit serious emotional disorders, including anxiety, which is the most prevalent symptom. Depression and obvious emotional distress may be accompanied by suicidal thoughts. Acne patients, especially the most severe acne patients, require effective and safe treatment.

At present, the understanding of the pathogenesis of acne is constantly developing, and it is found through research that the onset of acne may also be affected by familial genetics, diet, stress, environment and other factors. As a chronic inflammatory disease affecting the pilosebaceous units of the skin, key pathogenic factors involved include complex interactions among multiple factors such as abnormal keratinization of the pilosebaceous ducts, dysregulation of sebaceous gland activity, abnormal microbial colonization, imbalance of hormone microenvironment, and dysfunction of congenital and adaptive immunity. As a world-recognized opportunistic pathogen of acne vulgaris, cutibacterium acnes (C.acnes) has attracted extensive attention from scientists. Close balance between cutibacterium acnes and skin microbiota plays a key role in acne pathogenesis. However, current antibiotics used clinically for acne treatment cannot specifically target the target microorganism. If we develop more specific and targeted therapeutic drugs or regimens for cutibacterium acnes (pathogenic bacteria of acne), this will provide promising candidate interventions for the clinical treatment of acne.

Cutibacterium acnes is an anaerobic lipophilic gram-positive bacteria with relatively slow growth. In the skin of healthy adults, cutibacterium acnes colonizes the pilosebaceous units on the face and upper body. These bacteria primarily reside in the deeper regions of hair follicles and pores. Sebum, cell debris and metabolic by-products of surrounding skin tissues are used as their main sources of energy and nutrients. Under normal circumstances, cutibacterium acnes coexists harmoniously with skin cells. However, sebum secretion increases when sebaceous glands in hair follicles are hyperactive (sebaceous gland hyperplasia) or hair follicles are blocked, which can lead to excessive growth and proliferation of cutibacterium acnes. Cutibacterium acnes can produce secretory or surface attachment factors that can interact with the host, such as Christie-Atkins-Munch Petersen (CAMP) factor with hemolytic activity, dermatan sulfate binding adhesive (DsA) with fibrinogen binding activity, porphyrin with pro-inflammatory potential, short-chain fatty acids and host tissue component degrading enzymes (such as lipase, sialidase and hyaluronidase). To sum up, an important cause of acne is that the overproliferation of cutibacterium acnes in the skin generating its virulence factors, proteases, adhesion molecules and the like leads to the immune inflammatory response of the host.

According to Guideline for Primary Treatment of Acne Vulgaris (2023 Edition), current treatment methods for acne mainly include topical medication, systemic medication, physical and chemical therapy, and the like. Topical medication mainly consists of tretinoin drugs and antioxidants, which can kill cutibacterium acnes, improve keratinization of pilosebaceous ducts, and dissolve microcomedones, comedones and the like. However, as topical medication is applied locally, it is difficult to fully cover all acne-affected areas. Oral medication mainly consists of antibacterial drugs and hormone drugs. Antibacterial and anti-inflammatory drugs being selected for cutibacterium acnes and inflammatory response may cause drug resistance. Tretinoin can also be orally administrated and should be limited to cases of severe acne. It has been reported that the risk of suicide increases six months after isotretinoin treatment. Hormonal drugs cannot be orally administrated in large doses for a long time, which will induce drug-induced acne and other adverse reactions. Physical and chemical treatments mainly include photodynamic therapy and red-blue light therapy, laser and intense pulsed light therapy, radiofrequency therapy, and chemical peeling therapy. Such treatments may also cause physical or chemical damage to other normal skin. Therefore, better treatment methods are still needed for acne.

CAMP factor is closely related to the pathogenesis of acne related to cutibacterium acnes. After the cutibacterium acnes CAMP 2 gene is knocked out, colonization of cutibacterium acnes can be significantly reduced, and the infiltration of local immune cells in a mouse acne model and an expression level of inflammatory cytokines in skin lesion tissues are reduced, thus determining that CAMP2 is an important candidate target for acne treatment. If a vaccine can be designed for cutibacterium acnes as an important component, the limitations of existing clinical treatment methods can be overcome, thereby mitigating acne severity, curbing production of inflammatory factors, and reducing probability of scar formation.

### SUMMARY

As an anaerobic bacterium, cutibacterium acnes originally "coexists harmoniously" with skin cells. However, the excessive sebum secretion on the face obstructs hair follicles, creating an anaerobic environment in which cutibacterium acnes excessively proliferates and then secretes a virulence factor Christie-Atkins-Munch-Peterson factor (CAMP factor).

In order to solve a problem that there is still a lack of effective drugs for preventing and treating acne diseases, the present invention provides a recombinant protein vaccine targeting the cutibacterium acnes CAMP virulence factor. The recombinant protein vaccine neutralizes a CAMP virulence factor by inducing immune responses such as production of antibodies in vivo and inhibits cutaneous inflammation response, thereby achieving the effects of preventing and treating acne.

Therefore, a purpose of the present invention provides a protein for preventing and/or treating acne, where the protein is a wild-type cutibacterium acnes CAMP protein or a mutant thereof. Another purpose of the present invention is to provide a vaccine including the protein for preventing and/or treating acne induced by acne bacillus.

In order to implement the above purposes, the present invention firstly provides a protein for preventing and/or treating acne, where the protein is a wild-type cutibacterium acnes CAMP protein (CAMP factor 2) with an amino acid sequence as shown in SEQ ID NO: 1; or
the protein is a protein variant having sequence identity to SEQ ID NO: 1 and same or substantially equivalent biological activity.

Preferably, the protein variant has at least 70%, 80%, 90%, 95%, 97%, 98%, 99% or greater sequence identity to SEQ ID NO: 1 and the same or substantially equivalent biological activity.

More preferably, the protein variant includes at least one of the following mutations on SEQ ID NO: 1:
(a) glycine (G), threonine (T), serine (S), and tyrosine (Y) at position 82;
(b) glycine (G), serine (S), threonine (T), and tyrosine (Y) at position 153;
(c) glycine (G), glutamine (Q), threonine (T), serine (S), and tyrosine (Y) at position 154;
(d) glycine (G), glutamine (Q), threonine (T), serine (S), and tyrosine (Y) at position 158;
(e) glycine (G), glutamine (Q), threonine (T), serine (S), and tyrosine (Y) at position 160; and
(f) glycine (G), glutamine (Q), threonine (T), serine (S), and tyrosine (Y) at position 161.

Most preferably, the amino acid sequence of the protein variant is obtained by substituting methionine (M) at the position 82 of the cutibacterium acnes CAMP factor 2 protein with glycine (G), and the amino acid sequence of the protein variant is as shown in SEQ ID NO: 3.

A nucleotide sequence encoding the cutibacterium acnes CAMP protein variant is as shown in SEQ ID NO: 4.

The present invention provides a protein precursor for preventing and/or treating acne. The protein precursor includes the protein for preventing and/or treating acne, where the protein is fused to a signal peptide and/or a protein tag; and preferably, the protein tag is selected from at least one of the following: a histidine tag, a thioredoxin tag, a glutathione transferase tag, a ubiquitin-like modifier protein tag, a maltose-binding protein tag, a c-Myc protein tag, an Avi tag protein tag, and a nitrogen source utilization substance A protein tag.

The protein for preventing and/or treating acne is further fused to a protease recognition site for excising the protein tag; and preferably, protease is selected from at least one of enterokinase, TEV protease, thrombin, a coagulation factor Xa, carboxypeptidase A, and rhinovirus 3c protease.

The present invention further provides use of the protein or the protein precursor in preparation of a drug for preventing and/or treating acne or acne induced or triggered by cutibacterium acnes. Preferably, the acne is induced by infection with cutibacterium acnes.

The present invention further provides a recombinant protein vaccine for preventing and/or treating acne. The recombinant protein vaccine includes the cutibacterium acnes CAMP protein or the protein precursor, and a pharmaceutically acceptable excipient or adjuvant component.

The adjuvant component is an immunologic adjuvant. Preferably, the immunologic adjuvant is selected from at least one of the following: aluminum salt, calcium salt, phytosaponin, phytopolysaccharide, monophosphate lipid A (MPL), muramyl dipeptide, muramyl tripeptide, squalene-based oil-in-water emulsion (MF59), recombinant cholera toxin (rCTB), GM-CSF cytokine, lipid, cationic liposomal material, CpG ODN (synthetic CpG including a nucleotide sequence with unmethylated cytosine and guanine dinucleotide as a core sequence), or TLR3 (Toll-like receptors 3 ligand).

The aluminum salt is selected from at least one of aluminum hydroxide and alum.

The calcium salt is tricalcium phosphate.

The phytosaponin is either QS-21 or ISCOM.

The phytopolysaccharide is astragalus polysaccharide (APS).

The lipid is selected from at least one of the following: phosphatidylethanolamine (PE), phosphatidylcholine (PC), cholesterol (Chol), and dioleoylphosphatidylethanolamine (DOPE).

The cationic liposome material is selected from at least one of the following: (2,3-dioleoyloxypropyl) trimethyl ammonium chloride (DOTAP), N-[1-(2,3-dioleoyl chloride) propyl]-N,N,N-trimethylamine chloride (DOTMA), cationic cholesterol (DC-Chol), dimethyl-2,3-dioleyloxypropyl-2-(2-spermidinecarboxamido)ethyl ammonium trifluoroacetate (DOSPA), trimethyldodecylammonium bromide (DTAB), trimethyltetradecylammonium bromide (TTAB), trimethylhexadecylammonium bromide (CTAB), and dimethyldioctadecylammonium bromide (DDAB).

Preferably, the immunologic adjuvant is selected from at least one of an aluminum hydroxide adjuvant or a WGa01 adjuvant.

The vaccine is formulated as an intradermal injection or a subcutaneous injection, an intramuscular injection, an intravenous injection, an oral preparation or a nasal inhalation preparation.

Preferably, the vaccine is formulated as the intramuscular injection.

The present invention further provides use of the vaccine in preparation of a drug for preventing and/or treating infectious diseases induced by infection with cutibacterium acnes.

The present invention also provides a polynucleotide encoding the protein or the protein precursor.

Further, the nucleotide sequence of the polynucleotide is selected from at least one of SEQ ID NO: 2 or SEQ ID NO: 4.

The present invention also provides a recombinant vector including the polynucleotide.

Further, the recombinant vector is selected from at least one of an escherichia coli expression vector, an insect baculovirus expression vector, a mammalian cell expression vector, and a yeast expression vector.

Preferably, the escherichia coli expression vector is pET-30a.

Preferably, the insect baculovirus expression vector is pFastBacl.

Preferably, the mammalian cell expression vector is a CHO cell expression vector.

Further preferably, the CHO cell expression vector is pTT5 or FTP-002.

The present invention provides a host cell including the recombinant vector.

Further, the host cell is an escherichia coli cell.

The present invention further provides a preparation method for the protein for preventing and/or treating acne. The method includes the following steps: constructing a recombinant vector including a cutibacterium acnes CAMP protein or protein variant; transforming a host cell; expressing the protein; and purifying the protein.

Preferably, in the preparation method, the cutibacterium acnes CAMP protein or protein variant gene is inserted into a pET-30a (+) vector to construct an expression recombinant plasmid.

Preferably, in the preparation method, a prokaryotic expression system is adopted. More preferably, an escherichia coli expression vector system is adopted.

Preferably, in the preparation method, an inducer for inducing protein expression is isopropyl -β-D thiogalactoside, sucrose, and galactose. More preferably, the inducer is isopropyl-β-D thiogalactoside (IPTG).

Preferably, in the preparation method, a target protein is purified by an ultrafiltration membrane and an ion exchange process.

The present invention has the beneficial effects that the soluble cutibacterium acnes CAMP protein is expressed in escherichia coli by using the prokaryotic expression system. After non-reduced electrophoresis and reversed-phase high-performance liquid chromatography analysis, purity is over 95%. Electrophoresis analysis shows that the CAMP protein migrates at 25.3kDa, consistent with the theoretical molecular weight thereof. In the present invention, the recombinant protein vaccine is prepared by co-immunization with the purified CAMP protein and the adjuvant. The prepared recombinant protein vaccine has very high antigenicity and provides effective resistance against inflammation induced by cutibacterium acnes. In addition, the recombinant protein vaccine also can be used for treating or preventing inflammation induced by cutibacterium acnes. The vaccine can effectively induce the organism to produce cellular immunity and humoral immunity, and prevent acne symptoms induced by cutibacterium acnes. In the present invention, the preparation method for the recombinant protein vaccine is simple, and the quality of the recombinant protein vaccine facilitates control and large-scale production, so the recombinant protein vaccine is an alternative vaccine with potential clinical application value.

### BRIEF DESCRIPTION OF THE DRAWINGS

FIG. 1 shows an SDS-PAGE electrophoregram of a mutated cutibacterium acnes CAMP (CAMP Mut) protein during preparation in Embodiment 1.
FIG. 2 shows a molecular weight profile of the mutated cutibacterium acnes CAMP (CAMP Mut) protein detected by SDS-PAGE electrophoresis in Embodiment 1 (FIG. A shows a molecular weight detection diagram of the mutated CAMP protein; FIG. B shows purity of non-reduced SDS-PAGE electrophoresis; and FIG. C shows a spectrum of the mutated cutibacterium acnes CAMP (CAMP Mut) protein obtained by reversed-phase high-performance liquid chromatography analysis).
FIG. 3 shows a diagram of comparison results of hydrophobicity and hemolytic activity of a CAMP protein before and after mutation in Embodiment 2.
FIG. 4 shows a diagram of establishing a mouse acne model with high, medium and low doses of cutibacterium acnes in Embodiment 5.
FIG. 5 shows a trend diagram of binding antibody titers in a long-term immunogenicity experiment on NIH mice vaccinated with a CAMP recombinant protein vaccine in Embodiment 6.
FIG. 6 shows a result diagram of binding antibody titers and HE scores in a preventive experiment on a CAMP recombinant protein vaccine against infection with cutibacterium acnes in Embodiment 7.
FIG. 7 shows a diagram of acne diameter measurement and appearance score in a therapeutic experiment on a CAMP recombinant protein vaccine against infection with cutibacterium acnes in Embodiment 8.
FIG. 8 shows an appearance and histopathological diagram of acne lesions in the therapeutic experiment on a CAMP recombinant protein vaccine against infection with cutibacterium acnes, as well as a pathological HE score result chart in Embodiment 8.
FIG. 9 shows a result diagram of preventive administration of a wild-type CAMP and CAMP recombinant protein vaccine in Embodiment 9.

### DESCRIPTION OF THE EMBODIMENTS

Cutibacterium acnes CAMP factor is a secreted protein and pore-forming toxin, which is involved in the co-hemolytic activity of acid sphingomyelinase (ASMase) from other bacteria (such as staphylococcus aureus or host bacteria). When acting together with hemolysin of staphylococcus aureus, cutibacterium acnes CAMP 2 factor can also enhance hemolytic and cytolytic activity. The CAMP 2 factor exhibits potent cytotoxicity toward HaCaT keratinocytes and RAW264.7 macrophages, and can induce necrosis of sebocytes within sebaceous glands and trigger inflammation. Vaccinating mice with the CAMP factor can significantly reduce the growth of cutibacterium acnes and the production of MIP-2. The expression levels of the cutibacterium acnes CAMP factor and two pro-inflammatory factors (IL-8 and IL-1β) in acne lesions are higher than those in normal skin. The cutibacterium acnes CAMP factor is an important source of acne inflammation.

In other words, the CAMP factor is a cytotoxic secreted protein, which mainly acts on keratinocytes and macrophages and is an important factor to promote inflammatory response. The cutibacterium acnes CAMP factor can bind to immunoglobulins G and M and act as pore-forming toxin. Studies have shown that the CAMP factor and sphingomyelinase exert co-hemolytic activity, which can confer cytotoxicity upon keratinocytes and macrophages, and enhance virulence through degradation and invasion of host cells. In addition, the cutibacterium acnes CAMP factor can induce necrosis of sebocytes and trigger inflammation. In addition, some studies have shown that cutibacterium acnes specifically targets skin cells, namely, phagocytes such as keratinocytes and macrophages, and induces the production of pro-inflammatory cytokines, including IL-8, IL-1β, IL-12 and tumor necrosis factor-α, which leads to the inflammation in acne vulgaris.

Therefore, in the present invention, an anti-acne recombinant protein vaccine, a preparation method therefor, and an application thereof are designed. The anti-acne recombinant protein vaccine is prepared by the cutibacterium acnes CAMP factor 2 protein or a protein variant and a compound adjuvant.

In the present invention, a prokaryotic expression vector is used to express a recombinant protein in escherichia coli, and the soluble recombinant protein is obtained from a supernatant after lysis of bacterial cells. According to the physical and chemical properties of a prokaryotic expression system and the recombinant protein, after high-pressure lysis of bacterial cells, the supernatant is obtained and ultrafiltrated, and a target protein is obtained by both anion and cation exchange chromatography. The purified CAMP protein, along with an adjuvant, is administered for immunization.

Specifically, the applicant synthesized the gene sequence of the cutibacterium acnes CAMP protein or protein variant, and cloned the CAMP gene sequence into a pET-30a (+) vector. Recombinant plasmids were transformed into BL21 (DE3) competent cells, and positive monoclonal colonies were picked and expanded in culture. Then, an inducer isopropyl-β-D-thiogalactoside (IPTG) was added to induce a target protein expression; harvested bacterial cells were lysed at high pressure and the target protein was obtained from the supernatant after lysis of bacterial cells. Impurities were removed through ultrafiltration, and the target protein was subjected to anion-exchange and cation-exchange chromatography to obtain the target protein with high purity. The acne genetic engineering recombinant subunit vaccine was prepared by protein characterization, content determination, formulation with adjuvants, and other preparation methods. The cutibacterium acnes CAMP factor 2 protein (NCBI Reference Sequence: WP_002518322.1) was selected as the foundational recombinant protein for optimization. In order to improve the expression efficiency of the protein, the applicant removed a signal peptide region and selected a mature peptide segment (29-267) as an antigen.

In some embodiments of the present invention, polynucleotides having at least 60% sequence identity to SEQ ID NO: 2 and having the functions of the polynucleotides shown in SEQ ID NO: 2 also fall within the scope of protection of the present invention. Specifically, other polynucleotides with sequence identity include those obtained by the nucleotide sequence shown in SEQ ID NO: 2 by substituting, deleting or adding one or more (specifically, 1-50, 1-30, 1-20, 1-10, 1-5, 1-3, 1, 2 or 3) nucleotides, or those added with one or more (specifically, 1-50, 1-30, 1-20, 1-10, 1-5, 1-3, 1, 2 or 3) nucleotides to the N-terminus and/or C-terminus, with the function of the polynucleotide shown in SEQ ID NO: 2. Alternatively, other polynucleotides with sequence identity can be polynucleotides with 70%, 80%, 90%, 95%, 97%, 98%, 99% or greater sequence identity to SEQ ID NO: 2.

In some embodiments of the present invention, amino acids having at least 60% sequence identity to SEQ ID NO: 1 and having the functions of the amino acids shown in SEQ ID NO: 1 also fall within the scope of protection of the present invention. Specifically, other amino acids with sequence identity include those obtained by the amino acid sequence shown in SEQ ID NO: 1 by substituting, deleting or adding one or more (specifically, 1-50, 1-30, 1-20, 1-10, 1-5, 1-3, 1, 2 or 3) amino acids, or those added with one or more (specifically, 1-50, 1-30, 1-20, 1-10, 1-5, 1-3, 1, 2 or 3) amino acids to the N-terminus and/or C-terminus, with the function of the amino acids shown in SEQ ID NO: 2. Alternatively, other amino acids with sequence identity can be amino acids with 70%, 80%, 90%, 95%, 97%, 98%, 99% or greater sequence identity to SEQ ID NO: 2.

In some embodiments of the present invention, the protein variant includes at least one of the following mutations on SEQ ID NO: 1:
(a) glycine (G), threonine (T), serine (S), and tyrosine (Y) at position 82;
(b) glycine (G), serine (S), threonine (T), and tyrosine (Y) at position 153;
(c) glycine (G), glutamine (Q), threonine (T), serine (S), and tyrosine (Y) at position 154;
(d) glycine (G), glutamine (Q), threonine (T), serine (S), and tyrosine (Y) at position 158;
(e) glycine (G), glutamine (Q), threonine (T), serine (S), and tyrosine (Y) at position 160; and
(f) glycine (G), glutamine (Q), threonine (T), serine (S), and tyrosine (Y) at position 161.

In a most preferred embodiment of the present invention, artificial modification is performed in SEQ ID NO: 1, that is, methionine at the position 82 is mutated into glycine, and the gene and vector designed based on the modified amino acid sequence as shown in SEQ ID NO: 3 can implement a higher antigen expression level and reduce hemolytic activity of sheep red blood cells.

The amino acid sequence at positions 29-267 of the wild-type cutibacterium acnes CAMP factor 2 protein is shown as SEQ ID NO: 1.

SEQ ID NO: 1
MVEPTTTISATSTHELSASDARNSIQLLNAHIATLQSVQKSVPGSDYSDQIRDLLKAAFD
LRGLIETLAHGGIPFYDPSTIMPRIKLVATTIDTIHTATTTLQNKVRPAHVELGLEVTKAVLLTA
NPASTAKELDAEGAALKARLEKVSQYPDLTPNDVATVYVRTNFSKTIWQVRANRDRYILGH
KSAAVYKTLNHAITKAVGVRLNPKTTVGNIQAARTELLAAYQTAFNSPDVKKAA

The nucleotide sequence encoding the amino acid sequence as shown in SEQ ID NO: 1 is SEQ ID NO: 2.

ATGGTTGAACCGACCACCACCATCAGCGCGACCAGCACCCACGAACTGAGCGCGA
GCGACGCGCGTAACAGCATCCAGCTGCTGAACGCGCACATCGCGACCCTGCAGAGCGT
TCAGAAATCCGTTCCGGGCAGCGATTACTCCGACCAGATCCGTGACCTGCTGAAAGCGG
CGTTCGACCTGCGTGGTCTGATCGAAACCCTGGCGCACGGTGGCATCCCGTTCTACGAC
CCGTCCACCATCGGCCCGCGTATCAAACTGGTGGCGACCACCATCGATACCATCCACAC
CGCGACCACCACCCTGCAGAACAAAGTGCGTCCGGCGCACGTGGAACTGGGTCTGGAA

GTGACCAAAGCGGTGCTGCTGACCGCGAACCCGGCGTCCACCGCGAAAGAACTGGAC
GCGGAAGGTGCGGCGCTGAAAGCGCGCCTGGAAAAAGTTAGCCAGTACCCGGATCTGA
CCCCGAACGATGTTGCGACCGTTTACGTTCGTACCAACTTCTCTAAAACCATCTGGCAGG
TTCGTGCTAACCGTGATCGTTACATCCTGGGCCACAAATCTGCGGCGGTGTACAAAACC
CTGAACCACGCGATCACCAAAGCGGTTGGTGTTCGCCTGAACCCGAAAACCACCGTGG
GTAACATCCAGGCGGCGCGTACCGAACTGCTGGCGGCGTACCAGACCGCGTTCAACTCT
CCGGATGTTAAAAAAGCGGCGTAA

The sequence of CAMP protein (mutated protein CAMP Mut) is shown in SEQ ID NO: 3:

MVEPTTTISATSTHELSASDARNSIQLLNAHIATLQSVQKSVPGSDYSDQIRDLLKAAFD
LRGLIETLAHGGIPFYDPSTIGPRIKLVATTIDTIHTATTTLQNKVRPAHVELGLEVTKAVLLTA
NPASTAKELDAEGAALKARLEKVSQYPDLTPNDVATVYVRTNFSKTIWQVRANRDRYILGH
KSAAVYKTLNHAITKAVGVRLNPKTTVGNIQAARTELLAAYQTAFNSPDVKKAA

The nucleotide sequence encoding SEQ ID NO: 3 is shown in SEQ ID NO: 4:

ATGGTTGAACCGACCACCACCATCAGCGCGACCAGCACCCACGAACTGAGCGCGA
GCGACGCGCGTAACAGCATCCAGCTGCTGAACGCGCACATCGCGACCCTGCAGAGCGT
TCAGAAATCCGTTCCGGGCAGCGATTACTCCGACCAGATCCGTGACCTGCTGAAAGCGG
CGTTCGACCTGCGTGGTCTGATCGAAACCCTGGCGCACGGTGGCATCCCGTTCTACGAC
CCGTCCACCATCGGCCCGCGTATCAAACTGGTGGCGACCACCATCGATACCATCCACAC
CGCGACCACCACCCTGCAGAACAAAGTGCGTCCGGCGCACGTGGAACTGGGTCTGGAA
GTGACCAAAGCGGTGCTGCTGACCGCGAACCCGGCGTCCACCGCGAAAGAACTGGAC
GCGGAAGGTGCGGCGCTGAAAGCGCGCCTGGAAAAAGTTAGCCAGTACCCGGATCTGA
CCCCGAACGATGTTGCGACCGTTTACGTTCGTACCAACTTCTCTAAAACCATCTGGCAGG
TTCGTGCTAACCGTGATCGTTACATCCTGGGCCACAAATCTGCGGCGGTGTACAAAACC
CTGAACCACGCGATCACCAAAGCGGTTGGTGTTCGCCTGAACCCGAAAACCACCGTGG
GTAACATCCAGGCGGCGCGTACCGAACTGCTGGCGGCGTACCAGACCGCGTTCAACTCT
CCGGATGTTAAAAAAGCGGCGTAA

The solution of the present invention will be explained with reference to embodiments. It will be understood by those skilled in the art that the following embodiments are merely intended for illustrating the present invention, instead of limiting the scope of the present invention. If specific technologies or conditions are not specified in the embodiments, technologies or conditions described in literatures in the art or product specifications should be followed. If manufacturers are not indicated in reagents or instruments used, they are all conventional products that can be obtained through market purchase.

### Embodiment 1 Preparation of cutibacterium acnes CAMP protein

Based on NCBI database analysis and literature retrieval, a cutibacterium acnes CAMP factor 2 protein (NCBI Reference Sequence: WP5002518322.1) was selected as the foundational recombinant protein for optimization. In order to improve the expression efficiency of the protein, a signal peptide region was removed and a mature peptide segment (29-267) was selected as an antigen. An initial codon methionine (M) was added at the beginning of the mature peptide segment and was artificially modified, that is, methionine (M) at the position 82 was mutated into glycine (G). The gene and vector designed based on the modified amino acid sequence would achieve a higher antigen expression level and reduce hemolytic activity against sheep red blood cells.

The sequence of the mutated protein (CAMP protein), including 240 amino acids in full length, is designated as SEQ ID NO: 3.

MVEPTTTISATSTHELSASDARNSIQLLNAHIATLQSVQKSVPGSDYSDQIRDLLKAAFD
LRGLIETLAHGGIPFYDPSTIGPRIKLVATTIDTIHTATTTLQNKVRPAHVELGLEVTKAVLLTA
NPASTAKELDAEGAALKARLEKVSQYPDLTPNDVATVYVRTNFSKTIWQVRANRDRYILGH
KSAAVYKTLNHAITKAVGVRLNPKTTVGNIQAARTELLAAYQTAFNSPDVKKAA

The corresponding nucleotide sequence thereof is designated as SEQ ID NO: 4:

ATGGTTGAACCGACCACCACCATCAGCGCGACCAGCACCCACGAACTGAGCGCGA
GCGACGCGCGTAACAGCATCCAGCTGCTGAACGCGCACATCGCGACCCTGCAGAGCGT
TCAGAAATCCGTTCCGGGCAGCGATTACTCCGACCAGATCCGTGACCTGCTGAAAGCGG
CGTTCGACCTGCGTGGTCTGATCGAAACCCTGGCGCACGGTGGCATCCCGTTCTACGAC
CCGTCCACCATCGGCCCGCGTATCAAACTGGTGGCGACCACCATCGATACCATCCACAC
CGCGACCACCACCCTGCAGAACAAAGTGCGTCCGGCGCACGTGGAACTGGGTCTGGAA
GTGACCAAAGCGGTGCTGCTGACCGCGAACCCGGCGTCCACCGCGAAAGAACTGGAC
GCGGAAGGTGCGGCGCTGAAAGCGCGCCTGGAAAAAGTTAGCCAGTACCCGGATCTGA
CCCCGAACGATGTTGCGACCGTTTACGTTCGTACCAACTTCTCTAAAACCATCTGGCAGG
TTCGTGCTAACCGTGATCGTTACATCCTGGGCCACAAATCTGCGGCGGTGTACAAAACC
CTGAACCACGCGATCACCAAAGCGGTTGGTGTTCGCCTGAACCCGAAAACCACCGTGG
GTAACATCCAGGCGGCGCGTACCGAACTGCTGGCGGCGTACCAGACCGCGTTCAACTCT
CCGGATGTTAAAAAAGCGGCGTAA

A CAMP protein gene (restriction enzyme cutting site: NdeI/XhoI) was cloned into a pET-30a (+) vector to construct a plasmid pET-30a-CAMP. The plasmid was verified by restriction enzyme cutting and sequenced by an automatic sequencer. The vector was transformed into E.coli BL2 (DE3) competent cells after successfully constructed. After positive bacteria were cultured, an inducer IPTG was added for inducible expression, and a culture was collected by centrifugation to collect wet bacteria. The bacteria was suspended at a volume ratio of wet bacteria and buffer solution (20 mM PB, pH 7.5) of 1: 10, and was subjected to ultrasonic lysis or high-pressure lysis. After the cells were lysed, a supernatant was collected by centrifugation or membrane filtration. The supernatant was ultrafiltrated by an ultrafiltration membrane with the molecular weight cut-off of 100kDa-300kDa, then purified by anion exchange chromatography, and finally purified by cation exchange. The molecular weight and purity of the supernatant were analyzed by SDS-polyacrylamide gel electrophoresis, and the purity was analyzed by reversed-phase high-performance liquid chromatography (R-HPLC).

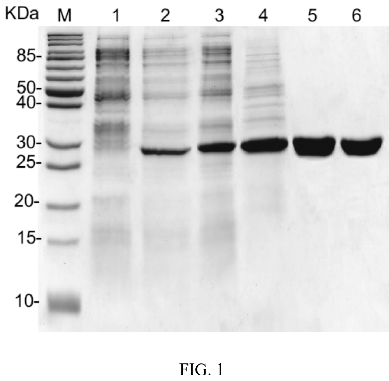

A target protein was expressed and purified through the expression vector constructed by gene cloning, yielding the CAMP protein with high stability and high expression level. Throughout the purification process, the purity of the target protein could be improved in each purification step, and the protein was relatively stable (see FIG. 1: M: protein molecular weight (Cat. No.: 26614, Thermo scientific^{™}); Lane 1: uninduced whole-cell lysate; Lane 2: induced whole-cell lysate; Lane 3: lysate supernatant; Lane 4: 300kDa ultrafiltration permeate/filtrate; Lane 5: anion exchange chromatography flow-through fraction; Lane 6: elution pool from cation exchange chromatography).

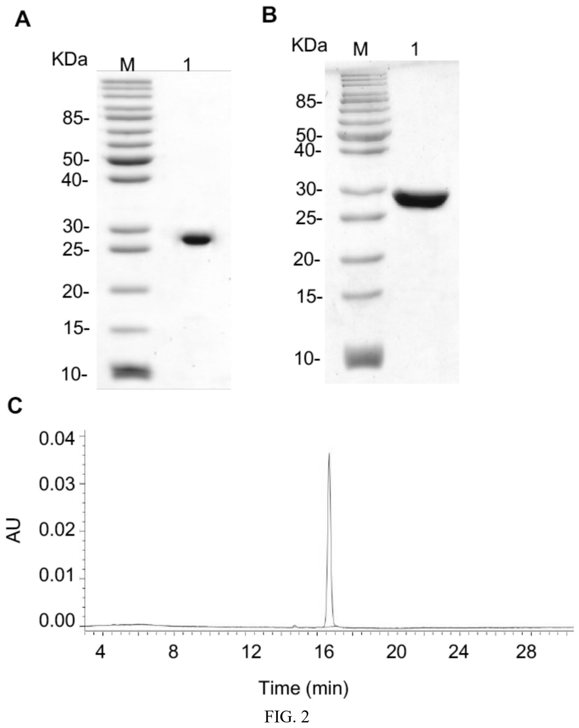

Reduced SDS-polyacrylamide gel electrophoresis shows that the calculated relative molecular weight of the target protein is 25,300 daltons and is basically consistent with the theoretical molecular weight thereof (FIG. 2A). In FIG. 2B-C, the purity of CAMP protein was detected by non-reduced SDS- polyacrylamide gel electrophoresis and reversed-phase high-performance liquid chromatography (R-HPLC) respectively, and the detection results show that the purity is more than 95%.

A preparation method for a wild-type CAMP protein (CAMP WT) is the same as that for the CAMP protein, except that the amino acid sequence of the gene cloned into the pET-30a (+) vector is shown in SEQ ID NO: 1 and a nucleotide sequence is shown in SEQ ID NO: 2.

### Embodiment 2 Hemolytic activity of cutibacterium acnes CAMP protein

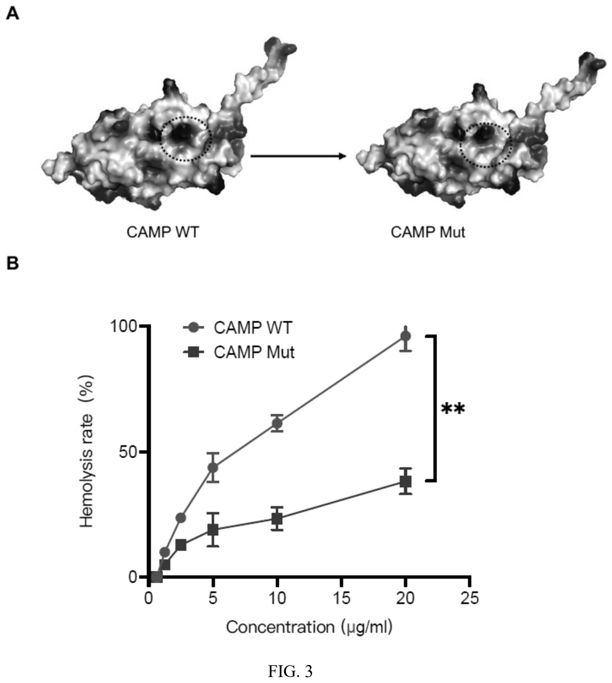

Purchased fresh sheep blood was centrifuged at 1000 × g for 5min at 4°C to collect red blood cells. Sheep red blood cells were washed with (0.01mol/L Na₃PO₄, 0.015mol/L NaCl, pH 7.0, PBS) for 3 times, and centrifuged at 1000 × g for 5min at 4°C to remove a supernatant each time. A total of 1mL sheep red blood cells were washed with a phosphate buffer (0.01mol/L Na₃PO₄, 0.015mol/L NaCl, pH 7.0, PBS) for three times, and centrifuged at 1000 × g for 5min at 4°C to remove the supernatant each time. Finally, 2% (V/V) red blood cell suspension was prepared with PBS, and pretreated for 30min with neurophospholipase having a final concentration of 50mU/. Subsequently, the CAMP protein and a wild-type CAMP protein were diluted to different concentrations. A total of 2% sheep red blood cell suspension was mixed with a diluted protein solution in an equal volume (200µl reaction system), incubated at 37°C for 1h, then placed at 4°C for 1h, and centrifuged at a low speed. Then, 100µl supernatant was taken. The optical density at the wavelength of 405nm was measured by an ELISA instrument, and a hemolysis rate was calculated. The results show that the hemolysis rate of CAMP decreases and is significantly statistical different from that before mutation (FIG. 3B). FIG. 3A shows a structural simulation diagram of the CAMP protein and the wild-type CAMP protein, and the results show that: the hydrophobicity of the N-terminal region of the CAMP protein is reduced compared with that of the wild-type CAMP protein. Blue represents hydrophobicity, and the larger blue area indicates the stronger hydrophobicity.

### Embodiment 3 Preparation of anti-acne recombinant protein (SEQ ID NO: 3) vaccine

The purified CAMP protein was detected for its concentration by an ultraviolet spectrophotometer, diluted to 0.5mg/mL by PBS, and filtrated aseptically for later use. Sterility test was performed according to the current Pharmacopoeia of the People's Republic of China, and endotoxin was detected by a gel method. Only when the endotoxin content was not higher than 10EU/mL, the protein could be used. The CAMP protein (SEQ ID NO: 3) was fully mixed/adsorbed onto an adjuvant.

Preparation of aluminum hydroxide adjuvant vaccine: phosphate buffer (10 mM PB, pH 7.0): 0.866g disodium hydrogen phosphate and 0.538g sodium dihydrogen phosphate monohydrate were dissolved in 800mL ultrapure water. After complete dissolution, the solution was brought to a final volume of 1L with ultrapure water. The solution was filtrated with a 0.22µm filter membrane. The phosphate buffer was added to an aluminum hydroxide adjuvant to prepare a mixture with the aluminum content of 0.84mg/mL, and the mixture was stirred at room temperature for 10min. Antigen dilution: a protein stock solution was diluted with the phosphate buffer to 500µg/mL. The aluminum content of the prepared vaccine was 0.42mg/mL, and the antigen content thereof was 250µg/mL. The mixture was stirred at room temperature for 1h after brought to a final volume, to obtain the vaccine formulated with the aluminum hydroxide adjuvant.

Preparation of WGa01 adjuvant vaccine: the protein stock solution was diluted to 500µg/mL with 20mM PB and 250mM NaCl, pH 7.5. WGa01 adjuvant and an antigen solution (500µg/mL) were mixed in an equal volume, resulting in the WGa01 adjuvant vaccine.

### Embodiment 4 Culture of cutibacterium acnes

Cutibacterium acnes was purchased from BNCC and cultured according to the operating instructions. Thioglycolate liquid culture medium and plates were incubated into an anaerobic incubator for 24h for deoxygenation. About 0.5mL liquid culture medium was aspirated and pumped into a freezing tube, fully dissolved, added into a testing tube containing 10mL culture medium, and mixed well. A total of 0.2mL bacterial suspension was aspirated and injected into the plate, and coated well. Such an operation was repeated twice to obtain two plates. After 3 days, a single bacterial colony was picked from the plate, subjected to anaerobic cultivation in a 100mL liquid culture medium at 37°C, and the colony morphology was found to be normal. Cultivation was continued until the broth became turbid, indicating that the bacteria had entered the logarithmic growth phase.

### Embodiment 5 Establishment of mouse acne model by cutibacterium acnes

Cultured cutibacterium acnes was transferred into a centrifuge tube, and centrifuged at 3000 × g for 5min. After a supernatant was removed, cells were washed twice with deoxidized PBS and suspended in the deoxidized PBS. Suspensions were prepared at low, medium, and high doses of 1*10⁸ cells/mL, 5*10⁸ cells/mL, and 1*10⁹ cells/mL using viable cells of cutibacterium acnes. With reference to the non-patent document Photochem Photobiol Sci.2006 Jan; 5(1):66-72, the number of viable bacterial cells of cutibacterium acnes was calculated based on the following assumption: the OD measurement value of 0.98-1.02 measured by a spectrophotometer at 550nm was corresponding to 5*10⁸ cells/mL. A total of 50µL bacterial suspension was intradermally injected into the abdominal skin of male ICR mice. On day 7, the animal was dissected, and the skin tissue was fixed in 4% paraformaldehyde to prepare paraffin sections.

Dewaxing the paraffin sections to water: the paraffin sections were sequentially placed into xylene I for 20min → xylene II for 20min → anhydrous ethanol I for 5min → anhydrous ethanol II for 5min → 75% alcohol for 5min for gradient dewaxing → washed with water. Hematoxylin staining: the paraffin sections were stained with hematoxylin for 3min-5min, and an excessive staining solution thereon was washed away with tap water. The paraffin sections were differentiated for several seconds with a 1% hydrochloric acid solution, rinsed with the tap water, turned blue with a 0.6%-0.7% ammonia solution, and finally rinsed with running water for several seconds. Eosin staining: the paraffin sections were dehydrated with gradient alcohol (85% ethanol → 95% ethanol) and stained in an Eosin staining solution for 5min. Dehydration and sealing: the paraffin sections were sequentially placed into anhydrous ethanol I for 5min → anhydrous ethanol II for 5min → anhydrous ethanol III for 5min → n-butanol for 5min → xylene I for 5min → xylene II for 5min until transparent, and the paraffin sections were taken out of xylene and slightly dried, and sealed with a neutral gum. Interpretation of paraffin sections: the cell nucleus is blue and the cytoplasm is red.

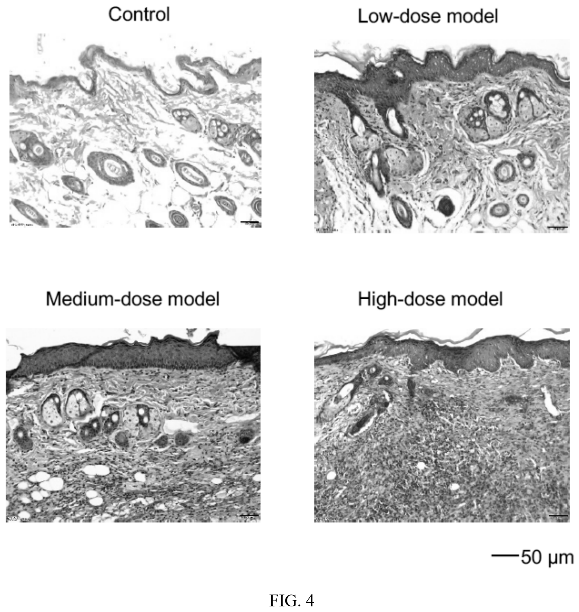

The results showed that after the injection of cutibacterium acnes in high, medium and low doses, local epidermis thickening, hyperkeratosis and acanthosis were observed. The pilosebaceous units were enlarged obviously, and keratinized substances could be observed. The infundibula of hair follicles were filled with keratinized substances and dilated into an ampulla-shaped structure. Capillaries in the upper dermis were obviously dilated and infiltrated with inflammatory cells, with subcutaneous tissue edema. Abscess was developed in the dermis, and blood vessels around the abscess were dilated and congested, and infiltrated with a large number of inflammatory cells (see FIG. 4).

### Embodiment 6 CAMP recombinant protein vaccine can induce long-term immunity in NIH mice

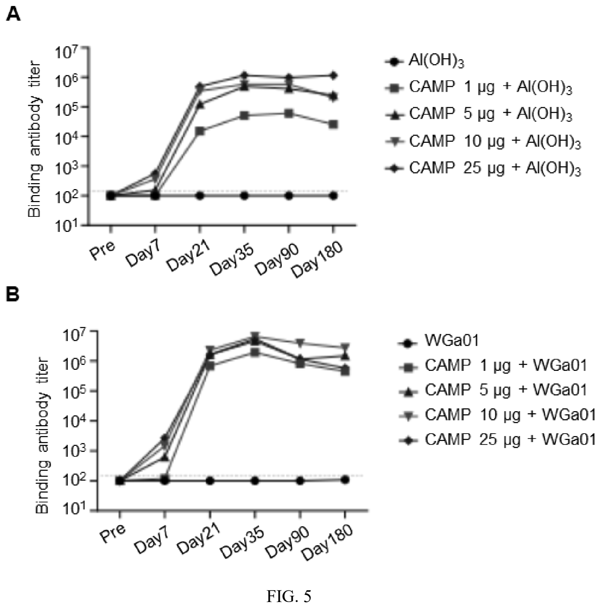

Six-week-old NIH mice were selected, and a WGa01 adjuvant and an aluminum hydroxide adjuvant were separately used to prepare a CAMP protein vaccine according to Embodiment 3. Each mouse was given three intramuscular injections in the thigh. Blood was collected from the posterior orbital venous plexus of the eye sockets of the mice or the eyeballs of the mice on days 7, 21, 35, 90, and 180 after the immunization, without anticoagulation. The blood was left at room temperature for 1h-2h, and then centrifuged at 1800 × g for 10min at 4°C to separate serum. The serum was stored at -20°C for later use. Experimental procedure steps are shown in FIG. 5. Detection of binding antibody titer: an antigen was diluted to a concentration of 1µg/mL with a coating solution (50mm carbonate coating solution), the diluted antigen was added into an ELISA plate at 100µL/well, and the plate was covered with a membrane and incubated at 2°C-8°C overnight; plate washing: the plate was washed 3 times with a washing solution (0.05% PBST), the washing solution was added into the plate at 300µL/well, and the residual liquid was removed; blocking: a blocking solution (1% BSA PBST) was added into the plate at 100µL/well, and the plate was covered with the membrane and incubated at 37°C for 1h; sample dilution: each sample was diluted with 1% BSA according to the corresponding dilution ratio and the diluent was added to the plate; plate washing: the plate was washed once with the washing solution (0.05% PBST), and the washing solution was added into the plate at 300µL/well, and the residual liquid was removed; sample addition: both quality control samples and test samples were added into the ELISA plate at 100µL/well, and the plate was covered with the membrane and incubated at 37°C for 1h; plate washing: the plate was washed 3 times with the washing solution (0.05% PBST), the washing solution was added into the plate at 300µL/well, and the residual liquid was removed; addition of test reagent: goat anti-mouse-IgG secondary antibody HRP was diluted 10,000 times with 1% BSA as the test reagent, the diluent was added into the plate at 100µL/well, and the plate was covered with the membrane and incubated at 37°C for 1h; plate washing: the plate was washed 5 times with the washing solution (0.05% PBST), the washing solution was added into the plate at 300µL/well, and the residual liquid was removed; color development: a TMB substrate color development solution was added into the plate at 100µL/well, and the plate was allowed to develop color at room temperature in the dark for 5min-10min; termination: a stop solution was added into the plate at100µL/well, to terminate the reaction; plate reading: an ELISA instrument was set to have a detection wavelength at 450nm (reference wavelength 630nm) and the OD value was read.

The results are as shown in FIG. 5A: when Al(OH)₃ adjuvant was used, 21 days after the first immunization, the serum specific binding antibody levels of mice in the CAMP dose groups of 1µg, 5µg, 10µg and 25µg all increased rapidly, and reached the peak on day 35. On days 90 and 180, the specific binding antibody was still at a high level.

The results are shown in FIG. 5B: when WGa01 adjuvant was used, specific binding antibodies were produced on day 7 after the first immunization of mice with CAMP recombinant protein in the dose groups of 1µg, 5µg, 10µg and 25µg. 21 days after the first immunization, the levels of serum specific binding antibodies in mice in each dose group increased rapidly, and reached the peak on day 35. 90 days after the first immunization, the antibody level was detected with no statistically significant decline, but it was still at a high level. On day 180, the specific binding antibody was still at a high level.

Conclusion: NIH mice are sensitive to the CAMP recombinant protein vaccine, which has good immunogenicity in mice and can induce strong functional immune antibodies. The long-term immunity in NIH mice can be induced by intramuscular injection of the CAMP recombinant protein vaccine for more than 180 days.

### Embodiment 7 Preventive effect of CAMP recombinant protein vaccine on infection with cutibacterium acnes

Six-week-old ICR mice were selected, and a vaccine was prepared with WGa01 adjuvant according to Embodiment 3. Each mouse was given three intramuscular injections in the thigh. 14 days after the last immunization, blood was collected from the posterior orbital venous plexus of the eye sockets of the mice without anticoagulation, placed at room temperature for 1h-2h, and centrifuged at 1800 × g for 10min at 4°C to separate serum, and the serum was stored at -20°C for later use. A binding antibody titer detection method was the same as that in Embodiment 6. One week after blood sampling, 50µL bacterial suspension (5*10⁸ cells/mL) was intradermally injected into the abdominal skin of male ICR mice. After 7 days, the skin tissues of the mice were dissected and fixed in 4% paraformaldehyde, and a subsequent HE staining experiment was performed in the same method as that in Embodiment 5.

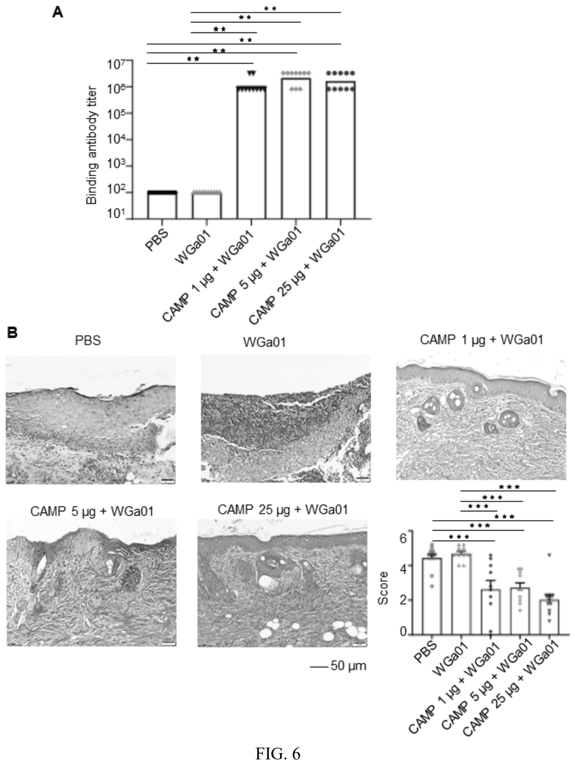

The results are as shown in FIG. 6: after each mouse was given two intramuscular injections in the thigh, the titers of CAMP-specific binding antibodies detected in the serum of ICR mice were all at relatively high levels in CAMP dose groups of 1µg, 5µg and 25µg (FIG. 6A). Compared with a model group, the level of inflammation in low, medium and high dose groups was significantly reduced (P<0.001), indicating a statistically significant difference (FIG. 6B). CAMP recombinant protein vaccine has a good anti-inflammatory effect on inflammation induced by cutibacterium acnes and can protect the skin to some extent.

### Embodiment 8 Therapeutic effect of CAMP recombinant protein vaccine on infection with cutibacterium acnes

Six-week-old ICR mice were selected, and 50µL bacterial suspension (5*10⁸ cells/mL) was intradermally injected into the abdominal skin of the ICR mice on days 0, 14, 28 and 42. The vaccine was prepared respectively with aluminum hydroxide and WGa01 adjuvant according to Embodiment 3, and each mouse was given three intramuscular injections in the thigh on days 0, 21 and 42. At weeks 2, 4, 6 and 8, the diameter of acne lesions was measured and the appearance score was given. On day 56, the mice were dissected, mouse skin tissues were fixed in 4% paraformaldehyde, and a subsequent HE staining experiment was performed in the same method as that in Embodiment 5.

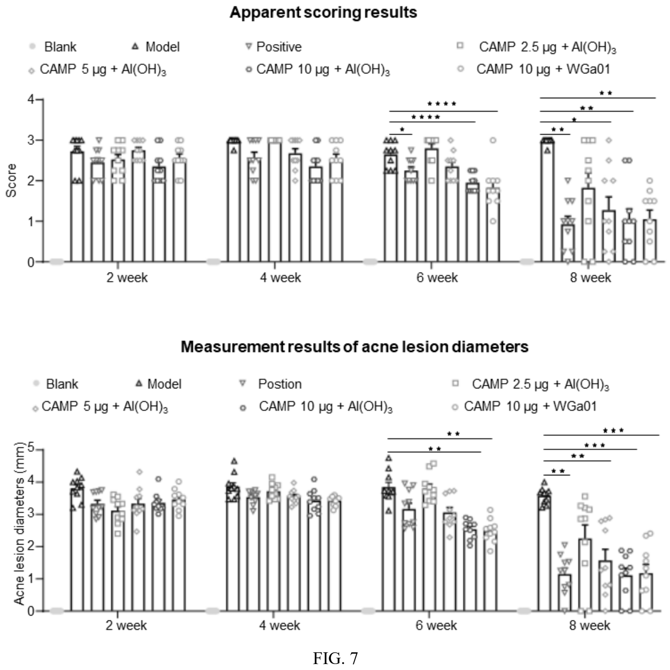

The results are as shown in FIG. 7: at week 6, compared with a model group, the diameters of acne lesions in a positive drug group (fusidic acid cream and tretinoin cream as positive drugs, drug combination, daily application) and 10µg CAMP protein (aluminum hydroxide adjuvant and WGa01 adjuvant vaccine groups) were significantly reduced, and the appearance scores were significantly reduced, indicating a statistically significant difference (P<0.05). At week 8, compared with the model group, the diameters of acne lesions in the positive drug group, 5µg and 10µg aluminum hydroxide adjuvant recombinant protein vaccine groups and 10µg WGa01 adjuvant recombinant protein vaccine group were significantly reduced, and the appearance scores were significantly reduced, indicating a statistically significant difference (P<0.05) (FIG. 7).

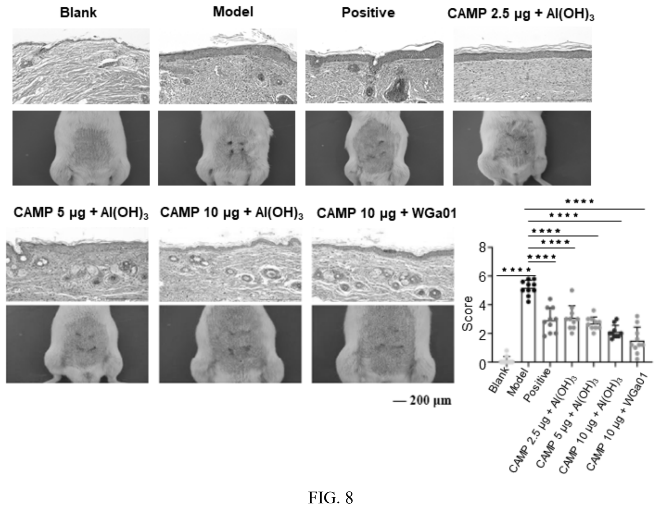

Compared with the model group, the histopathological scores of the positive drug group, the various doses recombinant protein vaccine groups with aluminum hydroxide adjuvant and the 10 µg recombinant protein vaccine group with WGa01 adjuvant were significantly reduced, indicating a statistically significant difference (P<0.0001) (FIG. 8).

### Embodiment 9 CAMP and wild-type CAMP recombinant protein vaccines have anti-inflammatory effects on infection with cutibacterium acnes

Six-week-old ICR mice were selected, and vaccines were prepared with WGa01 adjuvant and aluminum hydroxide adjuvant respectively according to Embodiment 3. Each mouse was given three intramuscular injections (10µg/mouse) in the thigh. After immunization, 50µL bacterial suspension (5*10⁸ cells/mL) was intradermally injected into the abdominal skin of male ICR mice. After 7 days, eyeballs were dissected and removed to take blood. The blood was centrifuged at 1800 × g for 10min at 4°C to separate serum after left at room temperature for 1h-2h, and a binding antibody titer detection method was the same as that in Embodiment 6. The skin tissues of the mice were fixed in 4% paraformaldehyde, and a subsequent HE staining experiment was performed in the same method as that in Embodiment 5.

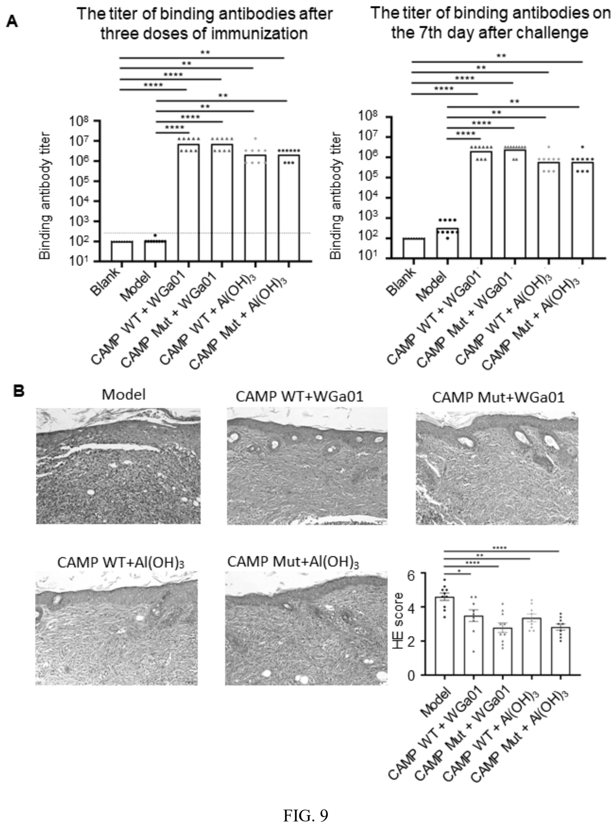

The results are as shown in FIG. 9: in the WGa01 adjuvant or the aluminum hydroxide adjuvant, the specific binding antibody titers of CAMP and wild-type CAMP recombinant protein vaccines were at a high level (FIG. 9A). Compared with a model group, the use of the WGa01 adjuvant, the aluminum hydroxide adjuvant, the CAMP and wild-type CAMP recombinant protein vaccines could both significantly reduce the level of inflammation induced by infection with cutibacterium acnes (P<0.05), indicating a statistically significant difference (FIG. 9B).

## Claims

1. A protein for preventing and/or treating acne, wherein the protein is a wild-type cutibacterium acnes CAMP protein with an amino acid sequence as shown in SEQ ID NO: 1; or the protein is a CAMP protein variant having sequence identity to SEQ ID NO: 1 and same or substantially equivalent biological activity.

2. The protein for preventing and/or treating acne according to claim 1, wherein the protein variant has at least 70%, 80%, 90%, 95%, 97%, 98%, 99% or greater sequence identity to SEQ ID NO: 1 and the same or substantially equivalent biological activity.

3. The protein for preventing and/or treating acne according to claim 1, wherein the protein variant comprises at least one of the following mutations on SEQ ID NO: 1:
(a) glycine (G), threonine (T), serine (S), and tyrosine (Y) at position 82;
(b) glycine (G), serine (S), threonine (T), and tyrosine (Y) at position 153;
(c) glycine (G), glutamine (Q), threonine (T), serine (S), and tyrosine (Y) at position 154;
(d) glycine (G), glutamine (Q), threonine (T), serine (S), and tyrosine (Y) at position 158;
(e) glycine (G), glutamine (Q), threonine (T), serine (S), and tyrosine (Y) at position 160; and
(f) glycine (G), glutamine (Q), threonine (T), serine (S), and tyrosine (Y) at position 161.

4. The protein for preventing and/or treating acne according to claim 3, wherein the amino acid sequence of the protein variant is obtained by substituting methionine at the position 82 of a wild-type cutibacterium acnes CAMP factor 2 protein with glycine, and the amino acid sequence is as shown in SEQ ID NO: 3.

5. The protein for preventing and/or treating acne according to claim 4, wherein a nucleotide sequence encoding a cutibacterium acnes CAMP protein variant is as shown in SEQ ID NO: 4.

6. A protein precursor, comprising the protein for preventing and/or treating acne according to any one of claims 1 to 5, wherein the protein is fused to a signal peptide and/or a protein tag; and preferably, the protein tag is selected from at least one of the following: a histidine tag, a thioredoxin tag, a glutathione transferase tag, a ubiquitin-like modifier protein tag, a maltose-binding protein tag, a c-Myc protein tag, an Avi tag protein tag, and a nitrogen source utilization substance A protein tag.

7. The protein precursor according to claim 6, wherein the protein for preventing and/or treating acne is further fused to a protease recognition site for excising the protein tag; and preferably, protease is selected from at least one of enterokinase, TEV protease, thrombin, a coagulation factor Xa, carboxypeptidase A, and rhinovirus 3c protease.

8. Use of the protein according to any one of claims 1 to 5 or the protein precursor according to any one of claims 6 and 7 in preparation of a drug for preventing and/or treating acne or acne induced or triggered by cutibacterium acnes.

9. The use according to claim 8, wherein the acne is induced by infection with cutibacterium acnes.

10. A recombinant protein vaccine for preventing and/or treating acne, comprising the protein according to any one of claims 1 to 5 or the protein precursor according to any one of claims 6 and 7, and a pharmaceutically acceptable excipient or adjuvant component.

11. The recombinant protein vaccine according to claim 10, wherein the adjuvant component is an immunologic adjuvant; and preferably, the immunologic adjuvant is selected from at least one of the following: aluminum salt, calcium salt, phytosaponin, phytopolysaccharide, monophosphate lipid A, muramyl dipeptide, muramyl tripeptide, squalene-based oil-in-water emulsion, recombinant cholera toxin, GM-CSF cytokine, lipid, cationic liposomal material, CpG ODN, or TLR3.

12. The recombinant protein vaccine according to claim 11, wherein the recombinant protein vaccine satisfies at least one of the following: the aluminum salt being selected from at least one of aluminum hydroxide and alum, the calcium salt being tricalcium phosphate, the phytosaponin being either QS-21 or ISCOM, the phytopolysaccharide being astragalus polysaccharide, the lipid being selected from at least one of the following: phosphatidylethanolamine, phosphatidylcholine, cholesterol, and dioleoylphosphatidylethanolamine, the cationic liposome material being selected from at least one of the following: (2,3-dioleoyloxypropyl) trimethyl ammonium chloride, N-[1-(2,3-dioleoyl chloride) propyl]-N,N,N-trimethylamine chloride, cationic cholesterol, dimethyl-2,3-dioleyloxypropyl-2-(2-spermidinecarboxamido)ethyl ammonium trifluoroacetate, trimethyldodecylammonium bromide, trimethyltetradecylammonium bromide, trimethylhexadecylammonium bromide, and dimethyldioctadecylammonium bromide; and preferably, the immunologic adjuvant is selected from at least one of an aluminum hydroxide adjuvant or a WGa01 adjuvant.

13. The recombinant protein vaccine according to any one of claims 10 to 12, wherein the vaccine is formulated as an intradermal injection or a subcutaneous injection, an intramuscular injection, an intravenous injection, an oral preparation or a nasal inhalation preparation; and preferably, the vaccine is formulated as the intramuscular injection.

14. Use of the vaccine according to any one of claims 10 to 13 in preparation of a drug for preventing and/or treating infectious diseases induced by infection with cutibacterium acnes.

15. A polynucleotide, wherein the polynucleotide encodes the protein according to any one of claims 1 to 5 or the protein precursor according to any one of claims 6 and 7; and preferably, the nucleotide sequence of the polynucleotide is selected from at least one of SEQ ID NO: 2 or SEQ ID NO: 4.

16. A recombinant vector, comprising the polynucleotide according to claim 15.

17. The recombinant vector according to claim 16, wherein the recombinant vector is selected from at least one of an escherichia coli expression vector, an insect baculovirus expression vector, a mammalian cell expression vector, and a yeast expression vector; preferably, the escherichia coli expression vector is pET-30a; preferably, the insect baculovirus expression vector is pFastBac1; preferably, the mammalian cell expression vector is a CHO cell expression vector; and further preferably, the CHO cell expression vector is pTT5 or FTP-002.

18. A host cell, comprising the recombinant vector according to claim 16 or 17; and further, the host cell being an escherichia coli cell.

19. A preparation method for the protein for preventing and/or treating acne, comprising the following steps: constructing a recombinant vector comprising the protein according to any one of claims 1 to 5 or the protein precursor according to any one of claims 6 and 7; transforming a host cell; expressing the protein; and purifying the protein.
